# EUROPEAN PATENT SPECIFICATION

(11) **EP 4 262 773 B1**
(45) Date of publication and mention of the grant of the patent: **26.03.2025**
(21) Application number: 21844208.5
(22) Date of filing: 17.12.2021
(51) Int. Cl.: A61K 31/167, A61K 31/19, A61P 19/00

(54) **A COMPOSITION COMPRISING LACTIC ACID FOR USE IN THE TREATMENT OF INTERVERTEBRAL DISC HERNIATION**
ZUSAMMENSETZUNG ENTHALTEND MILCHSÄURE ZUR VERWENDUNG BEI DER BEHANDLUNG VON BANDSCHEIBENHERNIEN
COMPOSITION COMPRENANT DE L'ACIDE LACTIQUE DESTINÉE À ÊTRE UTILISÉE DANS LE TRAITEMENT D'UNE HERNIE DISCALE

(30) Priority: 17.12.2020 SE 2051481
(43) Date of publication of application: 25.10.2023
(73) Proprietor: STAYBLE THERAPEUTICS AB, 411 33 Göteborg (SE)
(72) Inventor: LEHMANN, Anders, 421 66 Västra Frölunda (SE)
(74) Representative: AWA Sweden AB
(86) International application number: PCT/EP2021/086409
(87) International publication number: WO 2022/129476

(56) References cited:
- WO-A1-2015/140320
- US-A1- 2018 256 525
- NEBOJSA NICK KNEZEVIC ET AL: "Treatment of chronic low back pain - new approaches on the horizon", JOURNAL OF PAIN RESEARCH, vol. Volume 10, 1 May 2017 (2017-05-01), pages 1111 - 1123, XP055584504, DOI: 10.2147/JPR.S132769
- ANONYMOUS: "THE LATEST LACTIC ACID INJECTIONS FOR BACK PAIN... - BACK PAIN BLOG UK...", 8 June 2017 (2017-06-08), pages 1 - 2, XP055908818, Retrieved from the Internet <URL:https://backpainbloguk.com/2017/06/08/the-latest-lactic-acid-injections-for-back-pain/> [retrieved on 20220404]

## Description

### TECHNICAL FIELD

The present invention relates to a composition for use in the treatment of intervertebral disc herniation.

### BACKGROUND

Low back pain is thought to affect more than 80% of people at some point during their lifetime making it one of the most prevalent medical conditions worldwide. Low back pain is not a specific disease with known pathophysiology, but rather a symptom with many causes. Low back pain is the primary cause of disability in individuals under the age of 40. The lifetime prevalence of low back pain in the population is about 70-85% with about 10-20% experiencing chronic low back pain, being a major burden on the medical, social, and economic structures of essentially all countries.

Many patients who suffer from chronic low back pain have symptomatic bulging or herniated intervertebral discs (IVD), a condition known as disc herniation. An intervertebral disc is arranged between two adjacent vertebrae. The intervertebral disc is typically flexible and allows for motion between the adjacent vertebrae. It is formed by an outer portion in the form of a ring of connective tissue that mainly comprises collagen, and a semi-liquid central portion comprising e.g. collagen and proteoglycans. The outer portion is called annulus fibrosus (AF) and the central portion is called nucleus pulposus (NP). The NP is highly gelatinous with a composition of 70-90% water, 25-60% proteoglycan (dry weight) and 10-20% collagen (dry weight). The function of the NP is to sustain prolonged compression during the day and to resiliently re-inflate and reestablish disc height during the night. The NP is retained and surrounded by layers of cartilaginous AF. Together, the NP and the AF act as a resilient cushion. In the erect position, the weight of the body constantly compresses upon a stack of these cushions alternating between a series of vertebrae. During constant compression, the NP in each disc also behaves as a water reservoir, which is slowly and constantly being squeezed and drained of its water content through the end plates connected to the vertebrae. As a result, the disc height decreases slightly throughout the day. During bed rest, the weight of the body no longer compresses the disc. Due to the water absorbing nature of the NP, the flow of water then reverses from the vascular vertebrae back into the proteoglycan and collagen matrix. As a result, the disc height is reestablished and ready to provide support and flexibility for another day.

Disc herniation may be defined as deformation of the nucleus. Depending on the type and character of the deformation, herniation may be defined by disc bulging, disc protrusion, disc extrusion or disc sequestration. While the nucleus is contained by the outer annular fibers in a protrusion, that is not the case in an extrusion. The latter is also characterized by the neck of the herniation being narrower than the dome while the shape of a protrusion is more triangular. A disc extrusion is often followed by sequestration where free disc material is found in the spinal canal. Whereas an extrusion may be treated by intradiscal injections, sequestrations must be removed surgically.

Most disc herniations are found in people between 30 and 50 years old but it can occur in teenagers and older individuals as well. About 30% of all people in the Western World are affected by sciatica, mostly related to disc herniation, at some point of their lives. In Sweden, up to 2% of the population have surgery for disc herniation in their lifetime. Disc herniation occurs primarily in the lower back, i.e. in discs located between the lumbar vertebrae L1-L5. This type of IVD herniation is referred to as lumbar disc herniation (LDH). The two lowermost lumbar discs account for 95% of all cases of LDH. However, herniation may occur in other regions of the spine as well.

The annual incidence is between 0.5-2% of all adults and the disease is two times more common in males compared to females. Prevalence varies between 1-3%.

Many disc herniations are not treated at all since they disappear spontaneously. First-line treatment is non-surgical and includes both pharmacological and non-pharmacological therapy. The most commonly used drugs are NSAIDs but opioids are also frequently used. Antispasmodics such as baclofen are part of the pharmacological toolbox and while gabapentin and tricyclic antidepressants sometimes are used, although their efficacy is uncertain. Epidural steroid injections have been found effective in disc herniation. For a long time, physiotherapy was considered effective but more recent data are not supportive. Other non-pharmacological treatments include acupuncture and physical manipulation but evidence for their efficacy is weak.

Other therapeutic options are low-invasive and encompass chemonucleolysis or percutaneous nucleotomy.

During chemonucleolysis procedure an enzyme, ethanol or ozone/oxygen is injected into an intervertebral disc in order to dissolve the NP thereby reducing the pressure exerted by the NP of the intervertebral disc on e.g. a spinal nerve. To this end, intradiscal injection of chymopapain was widely used until the turn of the millennium. This treatment was found to be effective and the mechanism of action is enzymatic degradation of the extracellular matrix resulting in shrinkage of disc volume. Serious side-effects such as paraplegia (impairment of sensory and motor function of the legs) and anaphylactic reactions some of which were lethal later led to withdrawal of the product from the market. Another disadvantage of chemonucleolysis is that the treated disc may lose too much height, thus leading to back pain at a later stage caused by degeneration of the disc. Other enzymes such as condoliase have been tested/are under development but they too have the potential to produce various serious side-effects. Stem cells and platelet-rich plasma have also been evaluated and even if they show some promise, there is a paucity of controlled trials demonstrating their efficacy.

An alternative to chemonucleolysis is percutaneous nucleotomy, wherein the NP is partially removed mechanically or by vacuum such that the volume of the IVD is reduced. However, the amount of NP removed cannot be controlled in a precise way, leading to with unpredictable results and a low rate of success.

Surgery (discectomy) is the gold standard second-line therapy for disc herniation. Discectomy has a very good effect on symptoms, particularly radiating leg pain. Some 85% of patients who have had surgery for disc herniation are satisfied with the outcome. Nevertheless, discectomy is associated with a 15-25% risk of revision surgery.

Numerous postoperative complications can occur after a back surgery. The major ones are lumbar scarring and vertebral instability. The scar tissue extends and encroaches upon the laminectomy site and intervertebral foramen, leading to return of the pain, which leads to additional surgery. In fact, relapse operations are very common, reaching 10-20%. Unfortunately, the success rates of relapse operations are often below, in some cases far below the success rate of the first surgery. Relapse operations lead to more scarring and thus more pain. Currently, it is recommended to avoid surgical procedures unless the pain and inconvenience is absolutely unbearable. Even in cases of successful surgery providing long-term pain relief, the isokinetic test results clearly indicate weaknesses compared to individuals that have not been subjected to surgical procedures.

"The latest LA injections for back pain... - Back pain blog UK, June 8, 2017" is a blog post describing injection of lactic acid (LA) into discs of the spine. The author puts forward a theory that LA plumps up the disc and makes it stiffer, such that nerves do not get trapped and cause pain. Further, it is mentioned that injection of LA will hopefully trigger production of connective tissue and collagen, which will stiffen the disc.

WO 2015/140320 and US 2018/256525 describe a composition for use in the treatment of intervertebral disc (IVD)-related pain, such as low back pain, chronic low back pain, neck pain, chronic neck pain and coccygodynia. The composition may be administered in a therapeutically effective amount by a local injection to an IVD.

Knezevic et al., J Pain Res, 2017 May 10:10:1111-1123, doi: 10.2147/JPR.S132769 is a review article describing potential novel therapies that are on the horizon for the treatment of chronic low back pain. Among the therapies discussed are chemonucleolysis using chymopapain, and newer intradiscal therapies, including collagenase, chondroitinase, matrix metalloproteinases, and ethanol gel. Further, the use of subcutaneous monoclonal antibody acting as nerve growth factor called tanezumab is discussed. Finally, platelet-rich plasma and stem cells are being studied for the treatment of low back pain.

In view of the above-mentioned shortcomings, various less invasive surgical treatments for disc herniation have been developed. One such procedure is introduction of various devices, disclosed in e.g. US 5,800,550, WO 00/40159, WO 01/95818, and US 2004/097927, designed to fortify and immobilize the disc space between vertebrae into or in proximity of the IVD. However, these devices suffer from the disadvantage of reducing resilient cushioning, rotation, or mobility of the vertebrae, and of various post-surgical complications.

Despite extensive research in the area offering an increased amount of procedures available for treatment of disc herniation, there is still a need to provide a low-invasive procedure that is simple and that provides a long-term effect with minimal or no side effects.

### SUMMARY

In view of the above, the present invention aims to solve at least some of the problems of the prior art. To this end, the present invention provides a composition for use in the treatment of intervertebral disc herniation, wherein the composition comprises lactic acid and wherein the composition is administered into a disc space comprising the nucleus pulposus (NP) of a herniated intervertebral disc.

The term "treatment" in the context of the present application means both removal of cause and symptoms of disc herniation, as well as prevention of a possible relapse.

The term "intervertebral disc" (IVD) in the context of the present invention means an element lying between two adjacent vertebrae in the spine. Each intervertebral disc forms a cartilaginous joint to allow slight movement of the vertebrae and acts as a ligament to hold the vertebrae together. An intervertebral disc consists of an outer AF, which surrounds an inner NP. A human vertebral column comprises 23 intervertebral discs: 6 in the neck (cervical region), 12 in the middle back (thoracic region), and 5 in the lower back (lumbar region). In addition, intervertebral discs are also arranged between the coccygeal bones. An intervertebral disc may also be called a disc.

The term "NP" means the jelly-like substance in the middle of an intervertebral disc. The NP comprises chondrocyte-like cells, collagen fibrils, and proteoglycan aggrecans that aggregate through hyaluronic chains. Attached to each aggrecan molecule are the glycosaminoglycan (GAG) chains of chondroitin sulfate and keratan sulfate. The NP acts as a shock absorber and keeps the two adjacent vertebrae separated.

The term "AF" means a lamina of fibrous tissue and fibrocartilage formed as at the circumference of the NP. The AF serves to distribute pressure evenly across the intervertebral disc.

The term "disc space" means the space of an intervertebral disc which is filled by the NP and which has a circumference defined by the AF.

The term "cranial endplate" means the surface of an intervertebral disc facing towards the cranium. The cranial endplate is arranged on opposite side of the intervertebral disc compared to the caudal endplate.

The term "caudal endplate" means the surface of an intervertebral disc facing away from the cranium. The caudal endplate is arranged on opposite side of the intervertebral disc compared to the cranial endplate.

The term "facet joint" (also known as zygapophyseal joint) means a paired articular structure typically having a joint surface which is covered with articular cartilage. The facet joint is typically enclosed by a capsule. The facet joint form an articulation between the inferior articular process of the vertebrae and the superior articular process of the vertebrae. A facet joint is typically constructed to allow movement and to provide mechanical support to the vertebral column.

The term "transverse process" means a bony formation that extends laterally from the vertebral arch on both sides. It is also termed processus costarius.

By the term "disc herniation" is herein meant a deformation of the IVD such that the normal shape of the IVD is altered. Disc herniation may be nuclear herniation (disc bulge), disc protrusion, disc extrusion or sequestration.

By the term "flexion stiffness" is herein meant a characteristic describing the stiffness of an intervertebral disc arranged in a segment of a vertebral column. The flexion stiffness may be determined by applying a force to the segment of a vertebral column until it reaches a full lateral flexion mode, and by, thereafter, measuring the distance between the transverse processes of the vertebrae being arranged on the two opposite sides of the intervertebral disc, respectively. The full lateral flexion mode is defined as the state where the intervertebral disc of the segment of the vertebral column cannot be forced further without breaking of the segment of the vertebral column. This characteristic is measured in millimeter. The flexion stiffness is a way of characterizing the flexural rigidity of the segment of the vertebral column, and more specifically, the flexural rigidity of the intervertebral disc.

Flexural rigidity is generally defined as the force couple required to bend a non-rigid structure to a unit curvature. It is a measure of stiffness of a structural member; the product of modulus of elasticity and moment of inertia divided by the length of the member. In other words, it is the ratio of stress to strain in an elastic material when that material is being bent.

The concept of the present invention is to provide a composition for the treatment of IVD herniation in two steps. First, the herniated disc is dehydrated due to histological changes characterized by lysis of the extracellular matrix, which leads to reduction of both disc volume and disc height. The volume reduction is accompanied by pressure decrease which in turn leads to reduction of deformation of the disc. Thus, the protruding portion of the disc will be minimized or removed, leading to reduction of pressure on the nerves surrounding the disc, thus alleviating the pain. In the second step, the herniated disc treated by the composition of the present invention will undergo accelerated tissue remodeling thereby rendering the intervertebral disc stiffer, e.g. by transformation of the intervertebral disc into solid and dense connective tissue. The transformation of an intervertebral disc into solid and dense connective tissue makes it more stable, and consequently, the risk of a herniation relapse will be minimized. An intervertebral disc transformed into solid and dense connective tissue will furthermore not allow any nerve-irritating fluid component to leak out from the disc space, e.g. onto the outer surface of the AF and onto the spinal nerve roots. Since the pain associated with disc herniation is believed to result from a combination of nerve compression and leakage of nerve-irritating compounds, both of these symptom-generating factors will be reduced or eliminated by treatment of the IVD with the composition of the present invention.

The inventors of the present invention have surprisingly found that lactic acid seems to be successful in treatment of disc herniation. This finding is particularly surprising in view of the prior art rather focusing on decreasing the amount of lactic acid inside an intervertebral disc causing pain. For instance, US 2012/0022425 A1 discloses a method for reducing lactic acid within an intervertebral disc by injecting a lactic acid dehydrogenase inhibitor into the vertebral disc to inhibit production of lactic acid, and thereby alleviating back pain from lactic acid irritation. Further, WO 2013/092753 A1 reveals a compound of indole derivatives for inhibiting lactate production in the treatment of for example chronic back pain.

In view of WO 2015/140320, describing using lactic acid or a pharmaceutically acceptable salt thereof for reducing intervertebral disc-related pain by accelerating the ageing of an intervertebral disc thereby rendering the intervertebral disc stiffer, e.g. by transformation of the intervertebral disc into solid and dense connective tissue, an intuitive conclusion would be that the use of lactic acid in the treatment of disc herniation would be contraindicated since formation of connective tissue within the herniation may render it non-reducible and thus permanent. However, the inventors surprisingly found that due to stepwise and dual action of lactic acid, wherein histological changes characterized by lysis of the extracellular matrix accompanied by dehydration occurs initially, thus remedying the deformation, subsequently followed by alteration of the nucleus tissue to obtain fibrotic structure, thus stiffening and stabilizing the disc and preventing relapse, lactic acid is highly suitable for use in the treatment of disc herniation and prevention of re-herniation.

Lactic acid is a carboxylic acid having the following chemical structure:

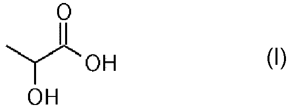

As may be seen in the formula (I) above, lactic acid comprises a chiral center on C-2. The two enantiomers of lactic acid are thus (S)-lactic acid (also known as L-(+)-lactic acid), and the other, its mirror image, is (R)-lactic acid (also known as D-(-)-lactic acid). A mixture of the two enantiomers in equal amounts is called DL-lactic acid, or racemic lactic acid. The term "lactic acid" in the context of the present invention means either of the enantiomers mentioned above or mixtures thereof. In other words, "lactic acid" in the context of the present invention may be in enantiomerically pure form or as a racemate.

Lactic acid may in an aqueous solution undergo deprotonation, i.e. lose a proton from its carboxyl group, producing the lactate ion CH₃CH(OH)COO⁻. The mole fraction of lactic acid to lactate ion is 1:1.

CH₃ CH(OH)COOH (aq) <→ CH₃ CH(OH)COO⁻ + H⁺ (I)

Lactic acid and lactate are naturally present in the human body.

The concentration of lactate ion in tissue water of a herniated IVD has been measured to be within the range of from 1 mmol/L to nearly 12 mmol/L, typically in the range of from 2 mmol/L to 6 mmol/L.

As seen in Table 1, the molecular weight of lactate ion is 89.07 g/mol. A molar concentration of 1 mmol lactate ion per liter tissue water in the intervertebral disc thus corresponds to a mass concentration of 89.07 mg/L. Similarly, a molar concentration of 12 mmol lactate ion per liter tissue water in the disc corresponds to a mass concentration of 1067 mg/L.

In a human, the disc space of a lumbar intervertebral disc has a volume estimated to be approximately from 1.5 ml to 3.0 ml.

In view of the above, the person skilled in the art could easily calculate the amount of lactate, expressed in moles or grams, in the disc. An example is given in Table 1.

**Table 1. Approximate amounts of lactate ion in a lumbar intervertebral disc of a patient with IVD herniation**

| | |
|---|---|
| Observed lactate ion concentration in the tissue water of a lumbar disc (L3-L4) of a patient with disc herniation | 1 - 12 mmol/L |
| Average volume of the disc space of a lumbar disc comprising the tissue water | 1.5 - 3 mL |
| **Calculated moles of lactate ion in the tissue water** | **0.0015 - 0.036 mmol** |
| Molar weight of lactate ion | 89.07 g/mol |
| **Calculated mass of lactate ion in the tissue water** | **0.134 - 3.21 mg** |

Naturally occurring lactic acid or lactate ion may interfere negatively with the function of the cells of the intervertebral disc, in particular the cells that produce the proteoglycans necessary for preventing the disc from ageing. Ageing of an intervertebral disc is initiated by a reduced supply of nutrients and oxygen via diffusion from the blood vessels primarily in the adjacent cartilaginous endplate. This will gradually induce an accumulation of metabolic waste products in the intervertebral disc, such as in the NP. One kind of metabolic waste product that may be present is lactic acid or lactate. Lactic acid may contribute to several mechanisms that will render cellular death in the intervertebral discs.

Lactic acid triggers events that lead to breakdown of large, water-binding molecules such as GAGs. In parallel, lactic acid stimulates liberation of TGF-beta, which in turn stimulates fibroblasts to produce collagen. The loss of water-binding capacity (dehydration) of the NP is followed by a reduction in volume.

Lactic acid may further liberate PGE₂ causing production of connective tissue such that stiffness of the disc is increased, which can be expressed as an accelerated ageing of the IVD.

Thus, an increase in the concentration of lactic acid in an intervertebral disc by administration of a composition comprising lactic acid into the disc space of the intervertebral disc would therefore have a dual and stepwise effect on the IVD, wherein histological changes characterized by lysis of the extracellular matrix lead to dehydration of the IVD, thus reducing the disc height, volume and pressure, and subsequently transformation of the NP into connective tissue.

As mentioned above, dehydration of the herniated IVD caused by the composition for use according to the present invention decreases the volume of the herniated IVD, which in turn decreases deformation and protrusion or extrusion of the herniated IVD. Subsequent accelerated controlled remodeling of the intervertebral disc, including transformation of the NP into connective tissue, renders the intervertebral disc stiffer, thus preventing disc herniation relapse.

Typically, the concentration of lactic acid may be increased in a herniated intervertebral disc, more specifically in the disc space, in order to dehydrate the IVD and subsequently accelerate the formation of fibrosis.

The inventors have found that a composition comprising lactic acid induces histological changes characterized by lysis of the extracellular matrix leading to dehydration of and thus decrease in volume of the herniated IVD, as well as a marked transformation of the intervertebral disc into connective tissue, thus making it stiffer. The volume decrease reduces the height of the IVD such that the herniation is removed or minimized. The marked transformation has been interpreted as an accelerated ageing of the intervertebral disc by transformation of the NP to connective tissue. Consequently, improvements for a patient with regard to disc herniation are achieved if a composition comprising lactic acid is administered into the NP of the herniated intervertebral disc resulting in an increased concentration of lactic acid inside the disc space.

Advantages of the composition for use in the treatment of disc herniation according to the present invention is a safer and more efficient treatment of disc herniation, further also being less expensive and less invasive than most treatments known in the state of the art. Further, lactic acid is biocompatible. The body of a vertebrate, such as a human, is capable of handling, such as degrading, lactic acid since this compound is naturally occurring in the body of the vertebrate.

The inventors suggest that when a composition for use in the treatment of disc herniation according to the present invention is administered into the NP, a dual and stepwise effect is obtained, as mentioned above. First, histological changes characterized by lysis of the extracellular matrix set in, at least partially dissolving the NP. As the extracellular matrix is composed of molecules providing high osmotic pressure, the IVD is dehydrated, which results in decreased volume and thus also decreased height of the IVD. This causes a pressure drop which is transmitted to the herniation. As a result, the herniation will shrink, thus alleviating the symptoms such as pain and limited range of motion. Finally, the NP in the disc space of an intervertebral disc is transformed to solid and dense connective tissue, similar to the connective tissue of the AF. The increased stiffness is expected to result in prevention of relapse of disc herniation and stabilization of the motion segment.

The composition for use of the present invention may be administered in an amount effective to increase the concentration of lactic acid in the disc space of a herniated IVD to a concentration higher than the concentration occurring during natural ageing. The composition for use according to the present invention is administered in an amount effective to increase the concentration of lactic acid in the disc space to at least above 20 mmol/L. The concentration of lactic acid in the disc space after administration of the composition for use according to the present invention may be from 20-25 mmol/L. Further, the concentration of lactic acid in the disc space after administration of the composition for use according to the present invention should be below 1,3 mol/L.

The composition for use according to the present invention may be administered in an amount effective to dehydrate the herniated intervertebral disc. By the term "dehydrate" is herein meant to reduce the water content in the NP. The water content may be decreased from 90% to 70%. As mentioned above, dehydration of the NP may be accompanied by volume reduction of the IVD. It should be noted that reduction in IVD height in the experiments described below was similar to the results obtained during chemonucleolysis using chymopapain or condoliase. Typically, the disc height is reduced by 5-20%, preferably by 10-15% as a result of administration of the composition for use according to the present invention. It should be noted that reduction in disc height due to natural ageing, associated with symptoms such as pain and limited range of motion is normally much higher, e.g. in the range of 30-50%. Therefore, the controlled and limited reduction in disc height caused by the composition for use according to the present invention being accompanied by transformation of the NP in the disc space of an intervertebral disc to solid and dense connective tissue is beneficial for treatment of disc herniation. The composition of the present invention thus causes a moderate reduction of disc height, thus alleviating discomfort and pain of the disc herniation, without being associated with a perspective of developing back pain at a later stage.

The composition for use according to the present invention may thus be administered in an amount effective to decrease the height of the herniated disc, and to initiate fibrosis of the previously herniated intervertebral disc.

The composition for use according to the present invention may have the concentration of lactic acid in the composition of at least 12 mmol/L, preferably from 50 to 12000 mmol/L, more preferably from 100 to 10000 mmol/L, even more preferably from 500 to 5000 mmol/L, most preferably from 800 to 2000 mmol/L.

The composition for use according to the present invention may be administered by local injection into the disc space comprising the NP of the herniated IVD. The local injection may typically be performed using a syringe under local or general anesthesia or under local anesthesia combined with sedation.

According to an embodiment, the amount of lactic acid in the composition administered in a single dosage within the range of from 2 mg to 1000 mg, such as from 5 mg to 500 mg, preferably from 10 to 300 mg, more preferably from 20 to 200 mg, more preferably from 90 to 180 mg. The single dosage corresponds to the amount of lactic acid being administered per disc space.

The composition for use according to the present invention may be administered at a single occasion or at repeated occasions in the single dosage.

By the term "single occasion" is herein meant at a single visit at a medical office, such as during a visit to the doctor e.g. at a hospital. The visit may be no longer than 24 hours, such as from 0.5 to 5 hours. The term typically, but not necessarily, implies that the single dosage is administered by only a single injection at the single occasion. However, the term also covers cases where the single dosage is administered at a single occasion but by several injections, such as from 2 to 10 injections per single occasion, e.g. from 2 to 5 injections per single occasion.

By the term "repeated occasions" is herein meant at more than one visit, i.e. a plurality of visits, at a medical office, such as during more than one visit to the doctor e.g. at a hospital. Each visit may be no longer than 24 hours, such as from 0.5 to 5 hours. The term typically, but not necessarily, implies that the single dosage is administered by only a single injection but at repeated occasions. However, the term also covers cases where the single dosage is administered at repeated occasions but by several injections, such as from 2 to 10 injections per each of said repeated occasions, e.g. from 2 to 5 injections per each of said repeated occasions.

The composition for use according to the present invention may be in the form of an aqueous solution comprising lactic acid in concentration mentioned above.

The composition for use according to the present invention may have a pH below 4.0, preferably below 3.5, more preferably below 3.0. Having low pH is beneficial since the IVD has a buffering effect, which may counteract the mechanism of the composition for use according to the present invention.

Typically, the composition for use in the treatment of disc herniation is provided in a formulation suitable for local injection of a therapeutically effective amount.

The composition for use according to the present invention may further comprise a contrast agent. The contrast agent may be an iodine-containing contrast agent, e.g. Visipaque, Omnipaque (iohexol) or the like. The contrast agent may be required for fluoroscopic guidance during the injection in order to confirm correct placement of the needle, as well as for a post-treatment radiologic examination, such as computer tomography (CT). The post-treatment radiologic examination may be performed in order to ensure that no leakage of the composition that has been administered to the IVD has occurred.

The disc herniation in the context of the present invention is selected from nuclear herniation (disc bulge), disc protrusion or disc extrusion.

In some examples, the composition may further comprise at least one agent selected from solubilizers, stabilizers, buffers, tonicity modifiers, bulking agents, viscosity enhancers, viscosity reducers, surfactants, cheating agents, preservatives, and adjuvants.

In an alternative example, a derivative of lactic acid may additionally or alternatively be administered as a pro-drug, such as ethyl lactate or polymers of lactic acid.

In a human, the amount of the composition to be administered may be within the range of from 0.05 mL to 5 mL, such as from 0.1 to 3 mL, e.g. from 0.2 mL to 2 mL. These amounts correspond more or less to the volume of the NP in a human. For a lumbar intervertebral disc, the amount of the composition to be administered may be approximately from 1.5 mL to 3.0 mL. For a cervical intervertebral disc, the amount of the composition to be administered may be approximately 0.5 mL. For a coccygeal intervertebral disc, the amount of the composition to be administered may be approximately 0.2 mL.

According to a second aspect, there is provided the treatment of disc herniation by administration of a therapeutically effective amount of lactic acid into the NP of an intervertebral disc of a patient in need thereof. Effects and features of this second aspect of the present invention are analogous to those described above in relation to the first aspect of the present invention.

According to a third aspect, there is provided use of lactic acid in the manufacture of a medicament for the treatment of disc herniation. Effects and features of this third aspect of the present invention are analogous to those described above in relation to the previous aspects of the present invention.

According to a fourth aspect, there is provided lactic acid for use in the treatment of disc herniation. Effects and features of this fourth aspect of the present invention are analogous to those described above in relation to the previous aspects of the present invention.

Further features of, and advantages with, the present invention will become apparent when studying the appended claims and the following description. The skilled person realizes that different features of the present invention may be combined to create embodiments other than those described in the following, without departing from the scope of the present invention.

### BRIEF DESCRIPTION OF THE DRAWINGS

These and other aspects of the present invention will now be described in more detail, with reference to the appended drawings showing embodiments of the invention, of which:
Fig. 1 depicts a cross section of a vertebral column of a human;
Fig. 2 shows a side view of two adjacent vertebras of a human vertebral column;
Fig. 3 illustrates a side view of a lower part of a vertebral column of a human;
Fig. 4 shows a herniated IVD;
Fig. 5 depicts different types of herniation;
Fig. 6 illustrates macroscopic appearance and quantification of sclerosis of the porcine NP after treatment with the composition for use according to the present invention;
Fig. 7 and 8 depict NP height and width, respectively;
Fig. 9 illustrates effect of the composition for use according to the present invention on the AF and NP; macroscopic appearance and quantification of sclerosis of the porcine NP after treatment with the composition for use according to the present invention;
Figs. 10 and 11 illustrate changes in T2-weighted MRI images after treatment with the composition for use according to the present invention.

### DETAILED DESCRIPTIONOF THE PRESENT INVENTION

The present invention will now be described hereinafter with reference to the accompanying drawings, in which exemplifying embodiments of the present invention are shown. The present invention may, however, be embodied in many different forms and should not be construed as limited to the embodiments of the present invention set forth herein; rather, these embodiments of the present invention are provided by way of example so that this disclosure will convey the scope of the invention to those skilled in the art. In the drawings, identical reference numerals denote the same or similar components having a same or similar function, unless specifically stated otherwise.

A vertebral column of a vertebrate comprises vertebrae, which surround and protect a spinal cord. In humans, the vertebral column is situated in the dorsal aspect of torso. Between two adjacent vertebrae, an intermediate intervertebral disc is arranged, i.e. the vertebrae are alternated by intervertebral discs forming the vertebral column. The specific structure and further parts of the vertebral column are known to a person skilled in the art.

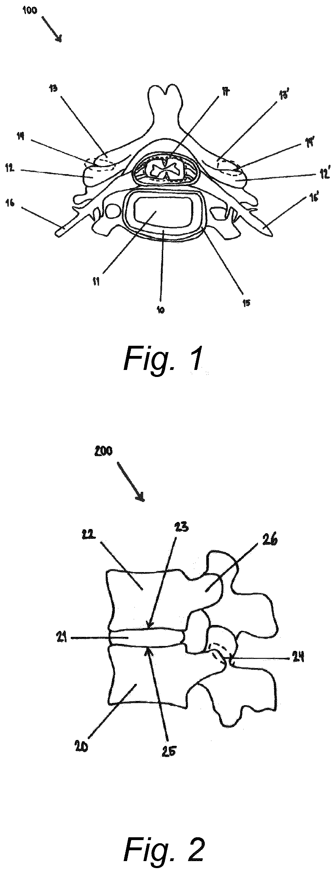

Fig. 1 schematically shows a cross section of a vertebral column 100 of a human. Adjacent to a vertebral body 15 of a vertebra, an intervertebral disc comprising an AF 10 and a NP 11 is arranged. The NP 11 fills up the so-called disc space of the intervertebral disc. The AF 10 surrounds the NP 11 and defines the border of the NP as well as of the disc space.

A spinal cord 17 is situated in the centre of the vertebral column, and adjacent to the intervertebral disc. Spinal nerves 16, 16', extend out from the spinal cord 17 to opposite sides of and closely to the intervertebral disc.

A facet joint 14, 14', is situated between an inferior articular process 13, 13' and a superior articular process 12, 12'. On opposite sides of the spinal cord 17, two facet joints 14, 14', are arranged, respectively. The facet joints 14, 14', are arranged in approximately the same cross-section and plane.

Fig. 2 schematically shows a segment of a vertebral column 200 comprising two adjacent vertebras 20, 22. A first vertebra 22 and a second vertebra 20 are arranged on opposite sides of an intervertebral disc 21. The first vertebra 22 is arranged relatively closer to the thorax, and the second vertebra 20 is arranged relatively closer to the sacrum. The caudal endplate 23 of the first vertebra 22 and the cranial endplate 25 of the second vertebra 20 are shown in Fig. 2. The cranial endplate 25 and the caudal endplate 23 are facing opposite sides of the intervertebral disc 21.

Fig. 2 also schematically shows how a facet joint 24 is arranged between the inferior articular process of the first vertebra 22 and the superior articular process of the second vertebra 20. A transverese process 26 extends laterally from the vertebral arch.

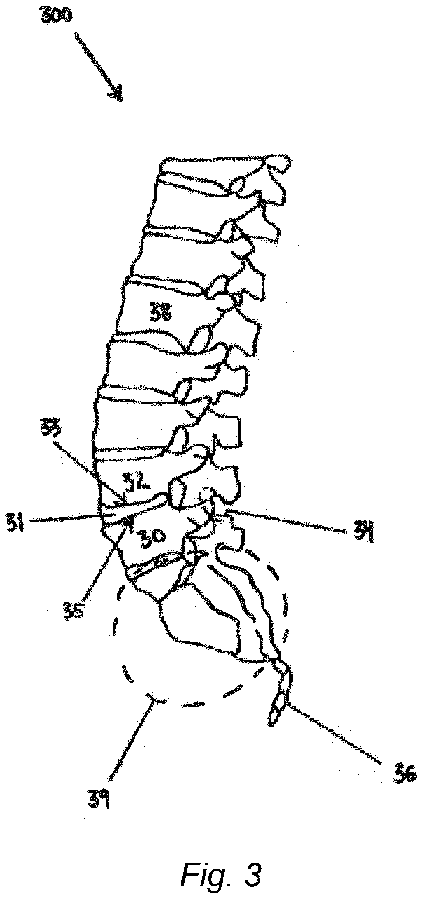

Fig. 3 schematically shows a lower part of a vertebral column 300. The coccygeal vertebrae 36 of the vertebral column is arranged at an end portion of the lower part of the vertebral column 300. The sacrum 39 of the vertebral column is arranged adjacent to the coccygeal vertebrae 36, closer to the thorax than the coccygeal vertebrae 36. A fifth lumbar vertebra, herein called L5, 30 is arranged adjacent to the sacrum 39, closer to the thorax than the sacrum 39. In a direction from the sacrum 39 towards the thorax, several vertebras are arranged in a row starting with L5, 30. Adjacent to the fifth lumbar vertebra 30, i.e. L5, the following vertebras are arranged in order: a fourth lumbar vertebra 32, i.e. L4, a third lumbar vertebra, i.e. L3, a second lumbar vertebra, i.e. L2, and a first lumbar vertebra 38, i.e. L1; the first lumbar vertebra being arranged relatively closest to the thorax. In between each two adjacent vertebras, an intermediate disc 31 is arranged. Intervertebral discs (not shown) are also imposing the coccygeal vertebrae 36.

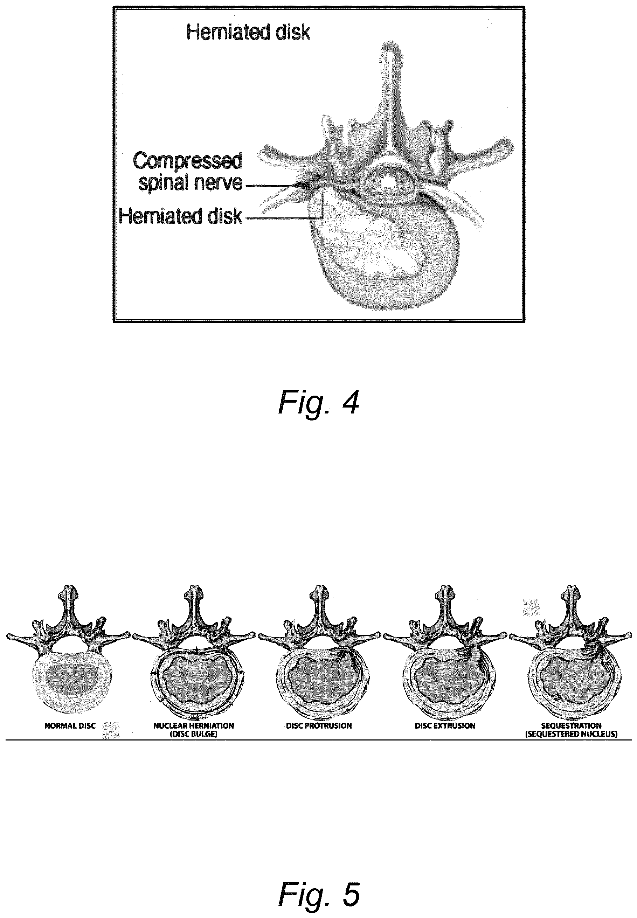

Fig. 4 depicts an example of a herniated IVD. As may be seen, during herniation the IVD is deformed such that the AF 10 is ruptured or weakened, allowing the NP 11 to protrude outside its normal boundaries. The protrusion may exert pressure on a spinal nerve, thus causing pain and limited range of motion. Fig. 5 illustrates different types of disc herniation.

### EXAMPLES

### Example 1

Three series of *in vivo* experiments were performed. The focus of the series differed slightly but the methodology was identical unless otherwise stated. Because of the different aims, measurements were not completely overlapping but the most important endpoint, sclerotization of the NP was quantified identically in all studies. For this reason, it was deemed justified to merge data from the different series.

When appropriate, Student's two-sided unpaired t test was used to compare the effects of LA with placebo treatment. The null hypothesis (no difference between LA and placebo) was rejected at p<0.05. Correlation between NP size measured using photographs and MR images was analyzed using Excel in Office 365.

LA was purchased from Merck Emprove (Darmstadt, Germany) and lohexol from Sigma Aldrich (St. Louis, MO, USA).

The pigs were pre-medicated by intramuscular injection of dexmedetomidin (Domitor^{®}vet, Orion Pharma, Sollentuna, Sweden) and a commercial mixture of zolazepam and tiletamine (Zoletil^{®}vet 100, Virbac, Carros, France) in conventional doses. Anesthesia was maintained by buprenophine (Vetergesic^{®}vet, Orion Pharma) 0.03 mg/kg intramuscularly, carpofen (Rimadyl^{®}vet, Orion Pharma) 4 mg/kg intravenously and isoflurane (Attane vet, VM Pharma AB, Stockholm, Sweden) using a Servo 900 respirator (Siemens, Munich, Germany). Before recovering from anesthesia, atipamezol (Antisedan^{®}vet, Orion Pharma) was given intramuscularly. On the first three days after surgery the pigs received oral carprofen 2 mg/kg twice daily.

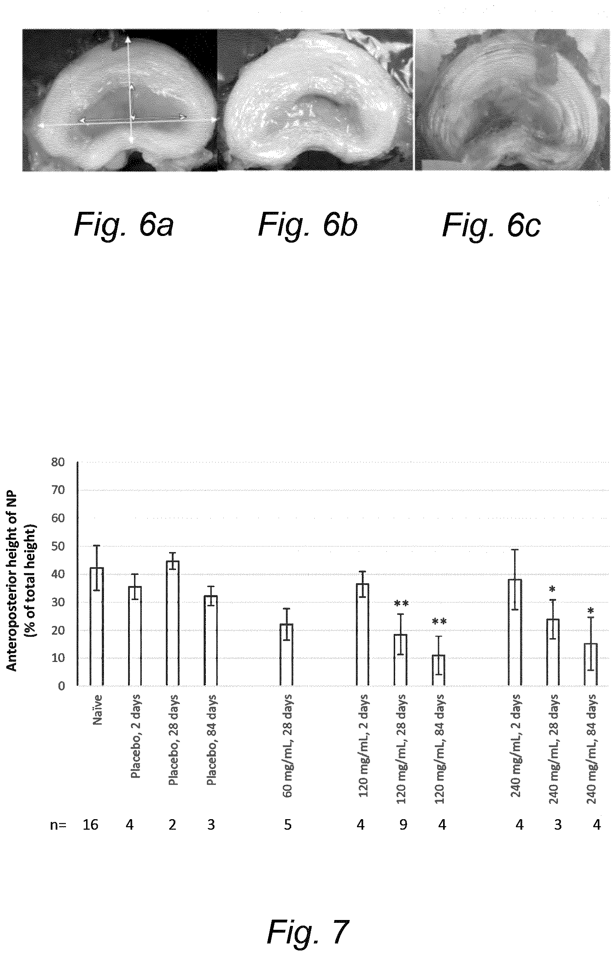

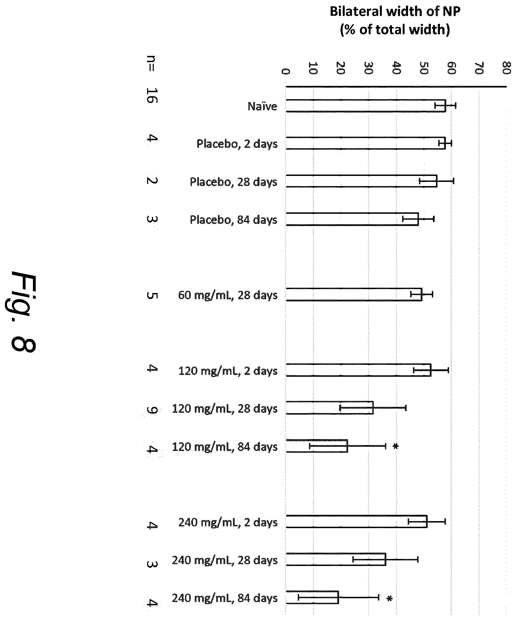

The pigs were placed on the right side. A 6 cm long incision was made from the costal arch to the iliac crest, just lateral to the lateral processes. The lumbar IVDs were approached by a retroperitoneal technique. The L3/4 IVD was incised and 0.2 mL of the LA-formulation (n=6) or the placebo (n=2) was injected under fluoroscopic guidance. The LA-formulation was composed of LA (120 mg/mL) and iohexol (180 mg I/mL) while placebo consisted of iohexol only. The concentration was determined based on the pilot experiments on the effects of LA on collagen secretion from human NP cells (as described in WO 2017/046030). In this set of *in vivo* experiments, the pH of the placebo formulation was adjusted to the same as the active formulation (about 1.5) using hydrochloric acid. The adjacent IVDs were not injected and served as negative controls. The volume of the pig NP is about 1 mL and it was estimated that 0.2 mL would be an appropriate injection volume. The height and width of normally appearing NP (Fig. 6a) in the anteroposterior (short vertical double arrow) and bilateral (short horizontal double arrow) directions, respectively, were measured and expressed in relation to the height (long vertical double arrow), and width (long horizontal double arrow) of the IVD. Note the reduction in NP size 28 (Fig. 6b) and 84 (Fig. 6c) days after LA treatment compared with the normally appearing NP (Fig. 6a). The group data for NP height as percent of total IVD height are shown in Fig. 7 and the corresponding data for NP width are illustrated in Fig. 8.

### Series 1

In the first pilot series, the objective was to determine if LA has the potential to sclerotize (induce fibrosis of) the IVD.

A total of 8 female pigs (mixed background of Yorkshire, Hampshire, and Landrace) with an average body weight of approximately 30 kg at surgery were used according to the procedure described above.

The animals were sacrificed 28 days after treatment and the spine from L1 to S2 was removed. The vertebral arches were removed from L1 to S1 to prevent interference with disc flexibility. The distances between the lateral processes of the vertebrae cranially and caudally to the injected IVD was measured with a caliper at full ipsi- or contralateral flexion. The difference of these distances was used as a measure of flexural rigidity. These measures were also documented for the untreated IVDs adjacent to the injected IVD in each lumbar spine. "Full flexion" was defined as the degree of flexion when stiffness is markedly increased at manual flexion at medium strength. The manual strength was not measured but the flexion was performed by two different individuals with identical results, which was considered sufficiently reliable to preliminarily demonstrate that the anatomical and histological changes seen indeed result in parallel biomechanical alterations.

Further, the IVD was cut in half and photographed. The bilateral width and anteroposterior height of normally appearing NP was measured and expressed as percent of the total IVD width and height, respectively, as depicted in Figs. 7 and 8.

The findings on NP space size from series 1 and 2 (see below) were merged since the procedures used, were identical.

There was a marked difference in flexural rigidity between the LA- and the placebo-injected IVDs as well as between the LA-injected IVDs and the 16 naïve IVDs (Table 2). There did not seem to be an effect on flexural rigidity of placebo injection as compared to untreated IVDs but the fact that only 2 pigs were injected with placebo precluded statistical verification.

**Table 2. Effects of LA on flexural rigidity of the isolated porcine lumbar spine**

| Treatment | Flexural rigidity (mm, mean±SD) | n | p |
|---|---|---|---|
| None | 8.1±1.4 | 16 | |
| Placebo | 8.2±1.8 | 2 | |
| LA, 120 mg/mL | 2.7±1.1 | 6 | <0.001 (none+placebo vs. LA) |

### Series 2

The aim of the second series (including 16 pigs) was to assess the safety and efficacy of the treatment and was part of the regulatory documentation required by the Swedish Medical Products Agency to initiate studies in humans. All procedures were as described above, with the following exceptions. The pH of the placebo formulation was not adjusted and since placebo and 2 doses of LA (0.2 mL of 120 or 240 mg/mL) and 3 survival times (2, 28 and 84 days) were to be evaluated, injections were done in 3 congruent IVDs in each pig to keep the number of animals within reasonable limits. In one group of animals, LA or placebo was applied onto the IVD outside the spinal foramen (external application) to assess any tissue damage resulting from leakage from the IVD or a misdirected injection. Due to this experimental design, spinal flexural stiffness was not measured in these pigs. The only other difference between the two series was that exposed tissues were evaluated histologically in the second but not first series.

The animals were grouped as shown in Table 3.

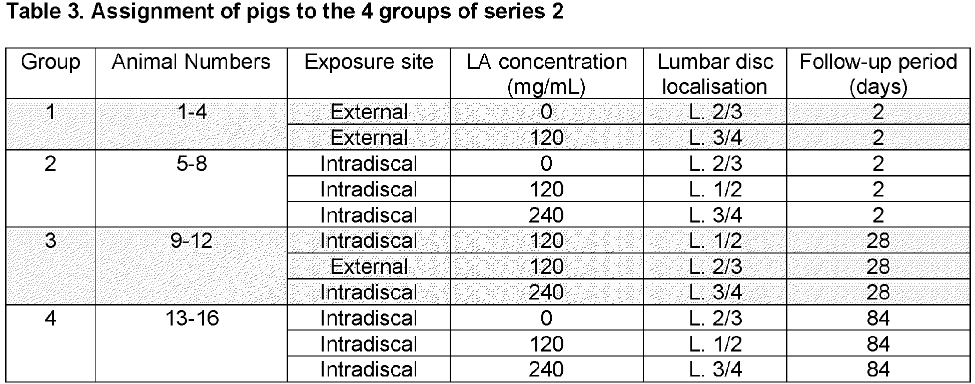

The lumbar spine was removed en bloc and any pathological changes were assessed macroscopically and photographed in extradiscal tissue onto which LA had been applied. Specimens of spinal nerves and muscle tissue were collected, fixed in 10% formalin, and processed for microscopy with hematoxylin/eosin staining. Injected IVDs were cut in half, inspected macroscopically, and photographed. Samples of the IVD were collected and processed for microscopy with hematoxylin/eosin. These turned out to include AF but very little NP so in order to obtain an improved overview of the IVD, the IVDs with adhering vertebrae were decalcified to enable preparation of axial sections of the whole IVD which were stained with Massons's trichrome.

### Series 3

Since effects of LA on the NP in the subsequent clinical study was planned to be assessed using MRI, the third series focused on the effects of LA (0.2 mL, 60 mg/mL) on sclerotization as measured by T2-weighted MRI. In addition, effects of LA on the expression of collagen I and II were studied by immunohistochemistry (IHC). The macroscopic changes after LA at 60 mg/mL were similar to those described above.

MR images were obtained using a 7T small animal MRI system (Bruker Biospec). A single 50 mm volume coil array was used (Tx/Rx). The dissected spines were stripped of the attached muscles, the spinous processes, transverse processes, and posterior aspects of the facet joints to allow them to fit in the MRI system. T2-weighted (TR: 2500-2834 ms, TE: 33-35.84 ms), 2D TurboRARE transversal and sagittal sequences were used. The TR and TE could not be maintained the same for all sequences due to required adjustments in field of view the different specimens, leading to an increased pixel matrix which affected the TR and TE. For the transverse sequences, a 500 µm slice thickness with 750 µm interslice distance and an in-plane resolution of 166x166 µm was used. For the sagittal sequences, a 750 µm slice thickness with 2000 µm interslice distance and an in-place resolution of 166x166 µm was used. In the transverse sequence of one of the pigs, the in-place resolution was 174x166 µm due to an increased field of view which could not be compensated for with a higher matrix size without significantly affecting TR.

Images were analyzed using Sante DICOM Viewer (version 8.1.5, Santesoft, Athens, Greece). The treated IVDs in all animals were assessed, and the IVD one level above the treated IVD was scanned as control.

The size of the NP was quantified using both MR images and photographs of the IVDs (see Fig. 6). The results from the macroscopic and MR analyses were then correlated.

### Immunohistochemical analysis of collagen I and II

The tissue was dehydrated in graded ethanol and embedded in paraffin. Sections were cut on a microtome, dewaxed, and incubated with polyclonal primary antibodies against collagen I (Abcam 34710) or II (Abcam 34712). The antibodies were diluted 250 (collagen I) or 200 (collagen II) times in phosphate-buffered saline containing 1% bovine serum albumin. Incubation was done at room temperature for 60 min. The sections were then incubated for 30 min at room temperature with horseradish peroxidase-conjugated secondary antibodies (Mach 2 Uni HRP, Biocare Medical) and immunoreactivity was visualized using diaminobenzidine (Biocare Medical).

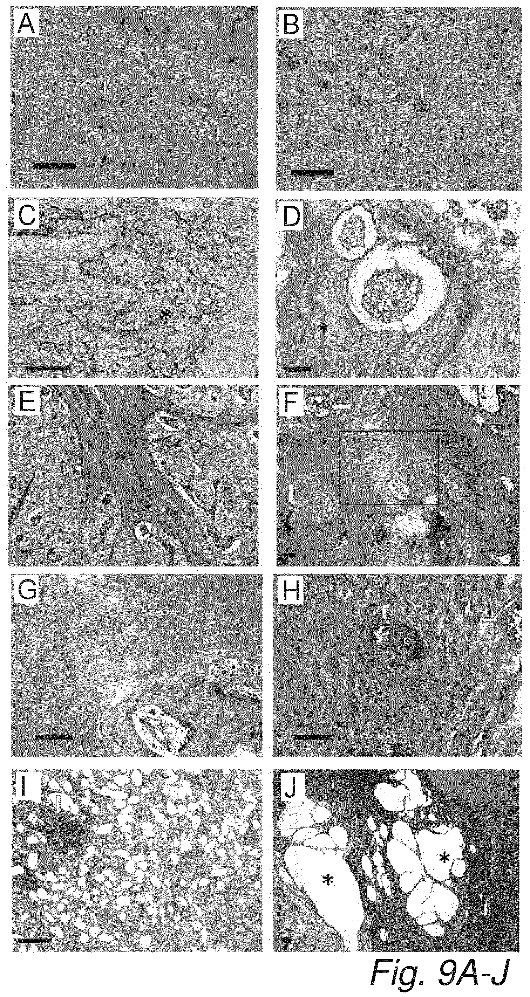

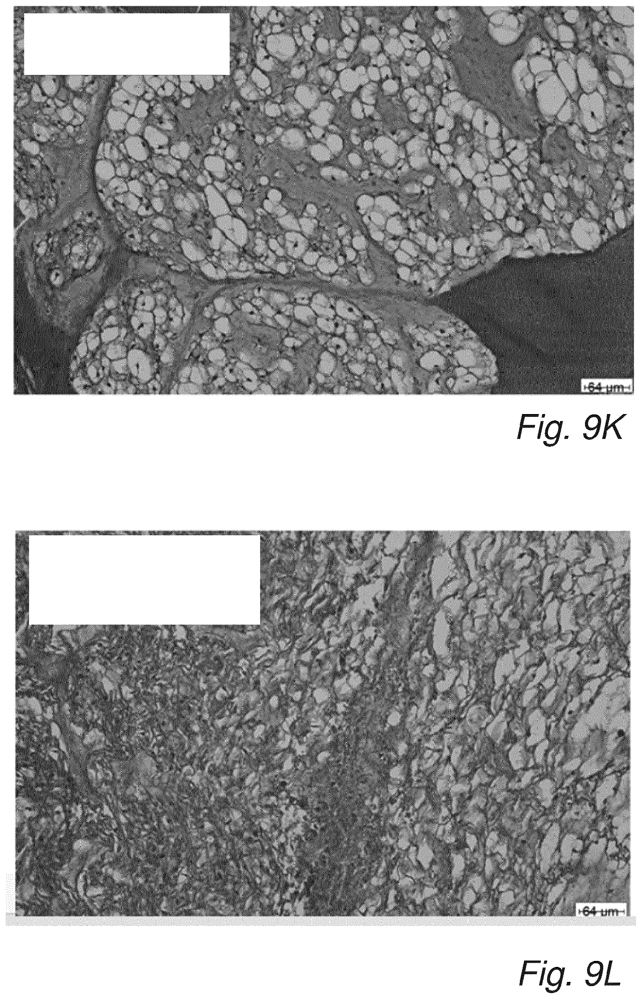

Fig. 9 illustrates effects of LA on the AF (A,B) and NP (C-J). LA or placebo (0.2 mL) was injected into IVDs of anesthetized pigs. The animals were sacrificed 4 or 12 weeks post injection and the IVDs were sectioned in half. After fixation in 10% formalin, decalcification and paraffin embedding, sections were cut at 5 µm and stained with hematoxylin&eosin (A,B) or Masson's trichrome (C-J). Fibrocartilagenous cells can be seen in the AF from untreated IVDs (arrows in A) but frequently, multicellular chondrons appeared after injection of LA (240 mg/mL, 12 weeks survival; arrows in B). NP of an untreated (extradiscal injection) IVD (C) consists of islets of notochordal cells (asterisk) embedded in weakly stained extracellular matrix. In LA-treated NP, collagenous fibers can be seen (asterisks in D; 120 mg/mL LA, 4 weeks survival and E; 120 mg/mL LA, 12 weeks survival). Occasionally, dense connective tissue with chondrocyte-like cells replaces the normal NP structure (F; 240 mg/mL LA, 12 weeks survival). Newly formed blood vessels (venules; arrows in F) and osteoid islets (asterisk in F) can be observed in the connective tissue. Boxed area in F is shown at higher magnification in G and newly formed blood vessels (arterioles, arrows) are shown in H. Cyst-like structures of varying size, possibly reflecting the "vacuum phenomenon", can be observed after LA injection after 4 (I; 240 mg/mL) and 12 (J, 120 mg/mL) weeks. Occasionally, small hemorrhages appear (arrow in I). Scale bar in all photomicrographs=100 µm.

Most histological analysis was done on the whole IVD sections stained with Masson's Trichrome both since these included central areas of the IVDs and also because fibrous tissue is more clearly visualized with Masson's than with hematoxylin&eosin staining.

At the two-day follow up period, hemorrhage and inflammatory changes were evident in occasional animals in which intradiscal injections had been done (not shown). The differences were not obviously more marked in animals that were treated with LA than in those injected with vehicle which suggests that these changes are associated with the procedure, rather than a specific LA effect. Similar findings were made in spinal nerves and skeletal muscle exposed to extraspinal LA (not shown).

Two days after injection with LA, there were marked histological changes characterized by lysis of the extracellular matrix (blue areas) of the NP and dissolution of notochordal cells (white areas with dark nuclei), as depicted in Fig. 9K (control) and 9L (injected with LA). As may be seen, both cells and matrix are in a state of lysis and degradation.

At the 28-day follow-up, there was a clear difference between the sites that had been treated with LA and those injected with placebo. Dense bundles of fibrotic tissue were seen in the animals that had been injected with LA (Fig. 9D). Similar to the macroscopic analysis, there did not appear to be any difference associated with the different concentrations of LA. No fibrotic changes were seen at the sites of extradiscal administration. In addition, residual hemorrhages, and cyst-like structures (Fig. 9I) were observed 28 days after LA injection. The nature of the latter is unknown, but they may represent the "vacuum phenomenon" according to findings in series 3.

At the 84-day follow-up, there were clear differences between placebo and LA treated IVDs. The histological changes were also more pronounced after 84 compared to 28 days. In LA injected IVDs, the following changes were seen in the NP: sclerosis (Fig. 9D-J), cystic degeneration (Fig. 9J), cartilaginous metaplasia (Fig. 9F, 9G), osteoid islands (Fig.9F, 9G) and reduction in the extracellular matrix. At 84 days, blood vessels appeared in the fibrotic tissue (Fig. 9H). There was no clear distinction in the degree of the changes between IVDs treated with the high or low dose of LA. The changes after LA treatment were not restricted to the NP since multicellular chondrons were observed in the AF (Fig. 9B).

The macroscopic changes after LA at 60 mg/mL were similar to those described above. These were reproduced on T2-weighted MRI where, as expected, the intensity of the sclerotized NP was much lower (Fig. 9B). The lamellar structure of newly formed connective tissue was also visualized by MRI. There was a close correlation (correlation coefficient=0.97) between the degree of sclerotization as estimated by analysis of photographs and MR images. Dark, mostly rounded areas were observed in 4 out of 5 LA-injected IVDs. They were pronounced in 2 IVDs and discrete (Fig. 9B) in the 2 others. These areas probably reflected the vacuum phenomenon and might be related to the large cyst-like structures seen microscopically.

Analysis of the disc height and disc width of treated and control IVDs showed that there was a very pronounced reduction in height but not width of the treated IVDs, as may be seen in Table 4. The relative disc height was expressed as a ratio of height of treated IVD/height of untreated (control) IVD. Analogously, the relative disc width was expressed as a ratio of width of treated IVD/width of untreated (control) IVD.

**Table 4. Relative changes in height and width between treated and control IVDs**

| Animal nr. | Relative disc height | Relative disc width |
|---|---|---|
| 1 | 0.2 | 1.01 |
| 2 | 0.33 | 0.99 |
| 3 | 0.7 | 0.99 |
| 4 | 0.36 | 1.04 |
| 5 | 0.55 | 1 |
| | | |
| Mean value | 0.43 | 1.01 |

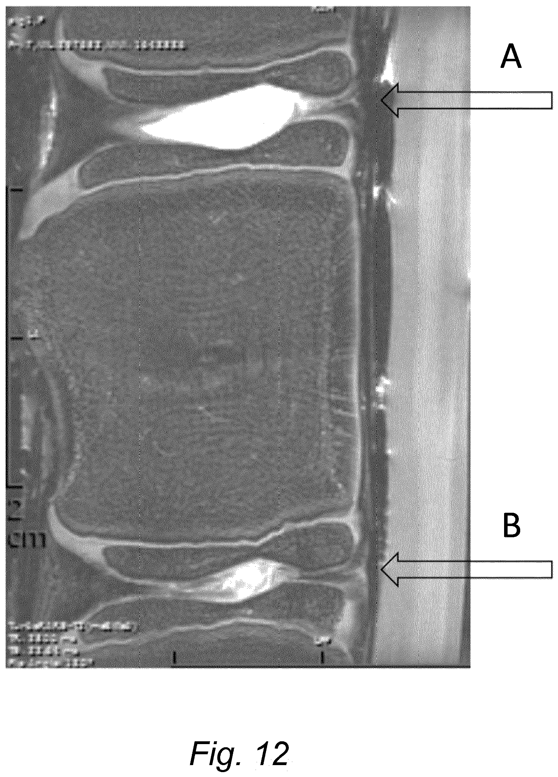

The effect is also confirmed as illustrated in the MR image shown in Fig. 12, wherein arrow A identifies an untreated control IVD, and arrow B identifies an IVD injected with 60 mg/ml LA 30 days prior to the image. As is evident from Fig. 12, a significant reduction in height of the treated IVD is observed.

In untreated IVDs, collagen I immunoreactivity was sparsely expressed in the NP but found at high levels in the AF. In contrast, collagen II immunoreactivity was located both in the NP and AF. There was no clear difference in collagen II immunoreactivity between treated and untreated IVDs but collagen I was strongly induced in the NP after LA treatment.

### Example 2

The study was a randomized, double-blinded, placebo-controlled, single ascending dose study with the primary objective to evaluate safety and tolerability following intradiscal injection of LA in Omnipaque or placebo (Omnipaque) in 15 patients with chronic discogenic low back pain. The secondary objective was to assess effects on the NP and disc height using T2-weighted MRI.

Scoring of visual analog scale (VAS) for both back and leg pain and Oswestry Disability Index (ODI) were used as exploratory objectives. The sample size was not based on power calculations but was considered appropriate for a single ascending dose study to provide initial safety data on 3 different dose levels of the LA formulation. The study has been registered on the ClinicalTrials.gov website and on the EU Clinical Trials Register. It was approved by the Stockholm Regional Ethics Committee (approval No. 2016-2323-31/4) and by the Swedish Medical Products Agency (approval No. 5.1-2016-86227).

Patients were recruited at Stockholm Spine Center (Upplands Väsby, Sweden) between April 2017 and August 2018.

Fifteen patients were randomized to either of the 3 dose groups. In each group, 2 patients were randomized to placebo and 3 to LA at 45, 90 and 180 mg (1.5 mL at 30, 60 and 120 mg/mL. The first 2 patients in each dose group were randomized to LA or placebo. Provided that no safety or tolerability concerns were identified within the first week (up to Visit 3) after administration (confirmed by a safety review committee consisting of 2 medical experts and a chairman without voting rights), another 3 patients received active treatment or placebo (2:1). Prior to dose escalation, the safety review committee assessed all safety data up to Visit 3 (1 week after treatment). In case of any safety or tolerability concerns, dosing could be discontinued, or the planned dose could be lowered.

The patient's total time in the study was up to 14 months. Each patient performed a screening visit within 60 days of planned treatment. An independent biostatistician at LINK Medical Research AB (Uppsala, Sweden) prepared a list of randomization numbers. Randomization was performed via an eCRF system (Viedoc^{™}) at least 5 working days before the planned treatment day to allow time for preparation of the investigational medical product (IMP). The test and reference IMPs were identical in appearance. The kits were labelled with the randomization number but did not contain any information on the identity of the formulation. This ensured that all staff at the clinics as well as the patients were blind to the treatment code. Treatment code envelopes were provided for each randomized patient. The code envelopes were kept in a secure place with limited access. In case of an emergency making it crucial for the investigator, or any other treating physician, to know whether the patient had received active formulation or placebo, the code envelope was to be opened. However, no such emergencies occurred and no treatment code envelope was opened.

The test product in this study was containing the active ingredient (S)-LA, which was provided to the site as a sterile solution in a syringe for single use. The LA doses administered were 45, 90 and 180 mg in a volume of 1.5 mL, corresponding to LA concentrations of 30, 60 and 120 mg/mL. The formulation for administration was prepared extempore and contained the contrast agent iohexol (Omnipaque^{™}) and water for injection. The final concentration of iohexol in the injected solution was 388 mg/mL. Small amounts of tromethamine, Na₂Ca edetate and HCl were also present. The solution was clear and colourless to slightly coloured.

LA (batch C16077AA) was manufactured, packed into vials and bulk labelled at Recipharm, Stockholm, Sweden. Other components for the IMP (Omnipaque [batch number 13407744] and water for injection) were purchased and released by Recipharm, Sweden. All components for the IMP were shipped to an extempore pharmacy laboratory as 1 kit per patient containing 1 vial of LA, 1 vial of Omnipaque, 1 vial of water for injection (active and placebo), together with preparation instructions and patient-specific labels that had been prepared by Recipharm. The final solution for injection was prepared aseptically by the extempore pharmacy laboratory (Apoteket AB Hospital Pharmacy, Uppsala, Sweden), packed into patient-specific syringes and labelled with "blinded" labels for each patient. The IMP prefilled and labelled syringes for each patient were sent to the clinic. If 2 injections were planned, 2 separate syringes were prepared (i.e. one syringe per IMP injection).

Matching placebo solution with identical appearance to the test product was used as reference treatment. The solution for administration was prepared extempore and contained the contrast agent iohexol (Omnipaque, batch number 13407744) diluted in water to a final iohexol concentration of 388 mg/mL and small amounts of tromethamine, Na2Ca edetate and HCl. An identical volume as for the test product (1.5 mL) was injected.

Since the study was too small to statistically evaluate effects of LA on the T2-weighted intensity of the NP, IVD height, VAS and ODI, only descriptive statistics were applied. All statistical analyses were performed using SAS^{®} version 9.4 (SAS Institute Inc., Cary, NC, USA). Results were presented by treatment group and in total where appropriate.

Continuous data were summarized using descriptive statistics, where the following parameters were reported: Number of patients with evaluable and missing observations, arithmetic mean, and standard deviation, median, first and third quartiles and minimum and maximum. Categorical data were presented as absolute and relative frequencies. When the absolute frequency was zero, the percentage was not presented. Unless stated otherwise, the denominator for percentage calculations was the total number of patients in the applicable analysis set, including patients with missing data. For variables with missing values, the number and percentage of patients with missing values was presented.

At the treatment day, patients received premedication with a sedative or anxiolytic and antibiotics, given as an i.v. infusion approximately 15 min before the intradiscal injection. The patient was placed in the right lateral decubitus position and injection was done using the two-needle technique under fluoroscopy guidance. Once correct intradiscal needle placement had been confirmed, a small volume (approximately 0.5 mL) of the formulation was first injected slowly (during half a minute) to verify that the distribution of the injectate was confined to the disc and that there was no leakage. If no leakage had occurred within half a minute, this procedure was repeated twice until the entire volume (1.5 mL) had been injected. If there was any leakage from the disc during injection, either after injection of a small volume or the entire volume (1.5 mL), the injection was interrupted. Immediately after treatment, patients had to remain in the prone position (alternatively in the lateral decubitus or supine position) for as long as possible (at least 4 hours after the last injection). All patients stayed overnight at the clinic after the injection, for observation and safety assessments. After leaving the clinic, patients were to be treated with analgesics and/or other measures according to standard clinical practice. They were also given advice on restricted physical activity during the first week.

Physical examination, blood pressure, heart rate, electrocardiogram (ECG), clinical chemistry and hematology were assessed using routine clinical methods. Pain during and 15 min after injection was measured using a VAS scale (0-100 mm) and the patient reported whether or not the pain reproduced the pain normally experienced in terms of location and type of pain. The distribution of Omnipaque within the IVD was documented according to the Dallas discogram scale. Local site reactions to the injection were noted, and AEs were graded for seriousness, intensity, and causality to the treatment. SAEs were defined according to the International Conference of Harmonisation guideline E2A.

All MRI investigations were performed using the same scanner (Siemens 1.5 T Avanto) with the following exceptions: Baseline MRI for one placebo-treated patient and 2 patients given the high dose was acquired in a Siemens Symphony 1.5 T scanner while all other scans were done in the Siemens 1.5 T Avanto scanner. Also, baseline and 3 month MRI were done in the Symphony scanner while the other scans were done in the Avanto scanner in 2 placebo-treated patients and one patient treated with the high dose. T1- and T2-weighted sagittal, coronal, and transversal sections were acquired at 4 mm before and after injection of gadolinium contrast.

Disc height measured as the maximal distance between 2 adjacent endplates and Pfirrmann grade were documented at screening and they were followed throughout the study. Since the Pfirrmann criteria are not sensitive enough to detect minor changes in disc hydration, changes in intensity of the NP at T2-weighted MRI were scored by 4 assessors blind to the group assignment of the patients. The assessors were instructed to score no change as "0" and obvious reduction in intensity as "1", regardless of time after treatment.

Patients reported their low back and leg pain level using a 0-100 mm VAS at the times indicated in Table 5. Disability was evaluated using the ODI.

**Table 5. Scoring of pain during injection and 15 min post injection**

| VAS pain injection site (mm) | STA363 45 mg (N=3) | STA363 90 mg (N=3) | STA363 180 mg (N=3) | Placebo (N=6) | Total (N=15) |
|---|---|---|---|---|---|
| Visit 2 (Treatment day), post-dose | | | | | |
| n/nmiss | 4/0 | 5/0 | 5/0 | 9/0 | 23/0 |
| Mean (SD) | 78 (10) | 48 (20) | 26 (36) | 27 (32) | **40** (33) |
| Median | 75 | 45 | 10 | 10 | 30 |
| Min, Max | 70, 90 | 30, 75 | 0, 90 | 5, 100 | 0, 100 |

| Visit 2 (Treatment day), 15 min | | | | | |
|---|---|---|---|---|---|
| n/nmiss | 4/0 | 5/0 | 5/0 | 9/0 | 23/0 |
| Mean (SD) | 20 (13) | 25 (27) | 9 (13) | 23 (22) | 20 (20) |
| Median | 20 | 30 | 0 | 10 | 15 |
| Min, Max | 4, 35 | 0, 65 | 0, 30 | 0, 70 | 0, 70 |
| n/nmiss = number of injections with evaluable/missing data, SD = standard deviation. | | | | | |

Pain intensity during the injection was higher in the 45 mg LA group compared to all other groups, but 15 min after the injection all groups reported similar levels of pain.

There were in total 24 AEs reported by 11 patients in the study (Table 6). All AEs were of mild-to-moderate intensity. The most common AEs were injection site pain (reported by all groups) and back pain (reported by all groups except the 60 mg/mL LA group). There were 16/24 AEs possibly or probably related to the treatment and 13 of those related to pain during or immediately after injection. All AEs were resolved within the 3-month follow-up period. There were no SAEs and none of the AEs led to patient withdrawal. During the extension phase, 6 AEs were reported by 4 patients and were judged as mild in intensity and with an unlikely relationship to the treatment.

**Table 6. Adverse events by severity and causality**

| | LA 30 mg/mL (n=3) | | LA 60 mg/mL (n=3) | | LA 120 mg/mL (n=3) | | Placebo (n=6) | | Total (N=15) | |
|---|---|---|---|---|---|---|---|---|---|---|
| | n (%) | m | n (%) | m | n (%) | m | n (%) | m | n (%) | m |
| Any adverse event | 2 (67%) | 4 | 3 (100%) | 3 | 3 (100%) | 8 | 3 (50%) | 9 | 11 (73%) | 24 |
| Any serious adverse event | 0 | 0 | 0 | 0 | 0 | 0 | 0 | 0 | 0 | 0 |

| Adverse events by severity | | | | | | | | | | |
|---|---|---|---|---|---|---|---|---|---|---|
| Mild | 1 (33%) | 2 | 1 (33%) | 1 | 3 (100%) | 6 | 2 (33%) | 3 | 7 (47%) | 12 |
| Moderate | 1 (33%) | 2 | 2 (67%) | 2 | 1 (33%) | 2 | 3 (50%) | 6 | 7 (47%) | 12 |
| Severe | 0 | 0 | 0 | 0 | 0 | 0 | 0 | 0 | 0 | 0 |

| Adverse events by causality | | | | | | | | | | |
|---|---|---|---|---|---|---|---|---|---|---|
| Unlikely | 2 (67%) | 3 | 0 | 0 | 2 (67%) | 3 | 2 (33%) | 2 | 6 (40%) | 8 |
| Possible | 0 | 0 | 0 | 0 | 1 (33%) | 1 | 2 (33%) | 4 | 3 (20%) | 5 |
| Probable | 1 (33%) | 1 | 3 (100%) | 3 | 2 (67%) | 4 | 2 (33%) | 3 | 8 (53%) | 11 |
| n = number of patients, m = number of events. | | | | | | | | | | |
| Percentages are based on the number of patients within each treatment group. | | | | | | | | | | |

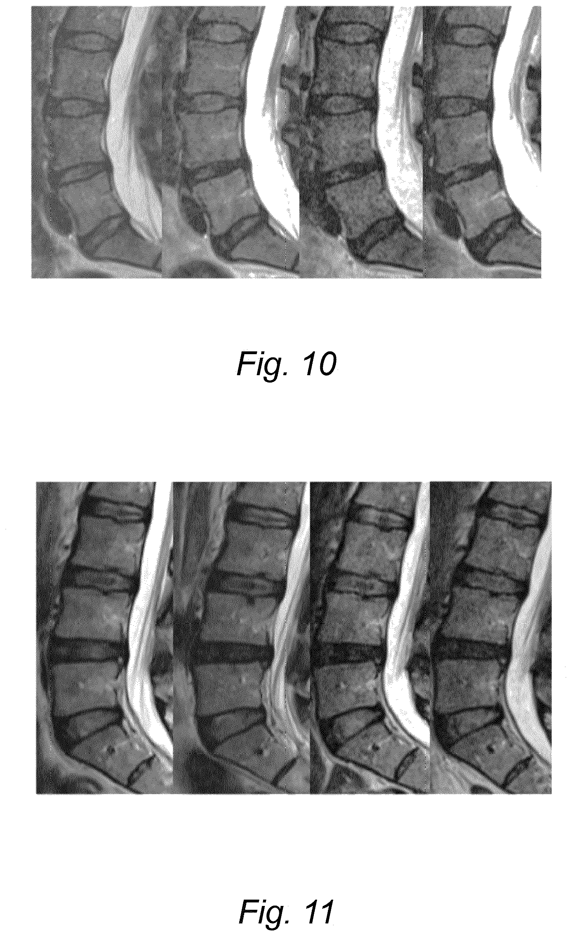

There was a tendency for a dose-response relationship with regard to posttreatment dehydration (Table 7). Two representative examples of changes in NP intensity after treatment with LA are presented in Figs. 10 and 11. Images in Fig. 10 are obtained from a patient treated with 60 mg/mL LA (L4/5 and L5/S1) and images in Fig. 11 are obtained from a patient injected with LA at 120 mg/mL (L4/5). The images (left to right) were acquired at screening and after 3, 6 and 12 months. Like the findings in pig IVDs, loss of intensity of the NP, probably reflecting sclerosis, often occurred in the periphery.

**Table 7. Changes in intensity of the NP at T2-weighted MR imaging**

| LA concentration (mg/mL) | NP intensity | | Baseline disc height (mm) | Disc height change from baseline (mm) | | |
|---|---|---|---|---|---|---|
| | | | | Mean±SD | | |
| | Mean+SD | Median | Mean+SD | 3 months | 6 months | 12 months |
| 0 | 0.2±0.4 | 0 | 9.8±1.3 | -0.2±0.7 | -0.5±1.4 | -1.0±1.4 |
| 30 | 0.4±0.5 | 0 | 9.5±1.7 | 0.3±1.3 | -0.5±0.7 | -0.5±0.7 |
| 60 | 1.0±0.0 | 1 | 9.4±0.9 | -1.0±0.7 | -0.8±0.8 | -1.2±0.8 |
| 120 | 0.9±0.3 | 1 | 11.2±1.6 | -1.2±1.1 | -1.4±1.1 | -1.8±1.3 |

Changes in intensity of the NP at T2-weighted MR imaging were scored as 1, clear reduction and 0, no change. The scoring was done by 4 assessors blind to the group assignment of the patients. Reductions in disc height were observed throughout the study (Table 8).

**Table 8. Individual scoring of change in NP intensity**

| **Patient No.** | **IVD** | **LA concentration** | **Assessor No.** | | | |
|---|---|---|---|---|---|---|
| | | | **1** | **2** | **3** | **4** |
| 1 | L5/S 1 | 0 | 0 | 1 | 1 | 0 |
| 2 | L4/51 | 0 | 0 | 0 | 0 | 1 |
| 2 | L5/S 1 | 0 | 0 | 0 | 0 | 1 |
| 3 | L4/5 | 0 | 0 | 0 | 0 | 0 |
| 4 | L3/4 | 0 | 0 | 0 | 0 | 1 |
| 4 | L4/5 | 0 | 0 | 0 | 0 | 1 |
| 5 | L5/S 1 | 0 | 0 | 0 | 0 | 0 |
| 6 | L4/5 | 0 | 0 | 0 | 0 | 0 |
| 6 | L5/S1² | 0 | 0 | 0 | 0 | 0 |
| | | | | | | |
| 7 | L3/4 | 30 | 0 | 0 | 0 | 0 |
| 8 | L4/5³ | 30 | 1 | 1 | 1 | 1 |
| 8 | L5/S1⁴ | 30 | 1 | 0 | 1 | 1 |
| 9 | L5/S 1 | 30 | 0 | 0 | 0 | 0 |
| | | | | | | |
| 10 | L3/4 | 60 | 1 | 1 | 1 | 1 |
| 10 | L4/5 | 60 | 1 | 1 | 1 | 1 |
| 11 | L4/5 | 60 | 1 | 1 | 1 | 1 |
| 11 | L5/S 1 | 60 | 1 | 1 | 1 | 1 |
| 12 | L5/S 1 | 60 | 1 | 1 | 1 | 1 |
| | | | | | | |
| 13 | L4/5 | 120 | 1 | 1 | 1 | 1 |
| 14 | L4/5 | 120 | 1 | 0 | 1 | 1 |
| 14 | L5/S 1 | 120 | 1 | 0 | 1 | 1 |
| 15 | L3/4 | 120 | 1 | 1 | 1 | 1 |
| 15 | L4/5 | 120 | 1 | 1 | 1 | 1 |

As may be seen, a clear reduction in disc height is achieved by administration of the composition for use according to the present invention.

It is evident that the composition for use according to the present invention dose-dependently reduces disc height (Table 9). Although the number of patients in the study is low, it is expected that dehydration of the NP is accompanied by deflation of the disc. The reduction in disc height is similar to that observed after chemonucleolysis (Table 9). There was no change in IVD width (not shown) demonstrating that IVD volume was attenuated.

**Table 9. Reduction in disc height provided by chemonucleolysis and the composition for use according to the present invention**

| | % decrease in disc height | | | |
|---|---|---|---|---|
| Dose of lactic acid (mg/mL) | 3 months | 6 months | 12 months | Chymopapain or condoliase |
| 0 | 2 | 5 | 10 | 16* |
| 30 | +3 | 5 | 5 | 30^{£} |
| 60 | 11 | 8 | 13 | 9^{$} |
| 120 | 11 | 12 | 16 | |

| | | | | |
|---|---|---|---|---|
| *7 years FU; Leivseth et al., 1999, £5 weeks to one year FU; height loss correlated with loss of T2 signal; Szypryt et al., 1987; $ one year FU; Chiba et al, 2018 | | | | |

It should be noted that even the highest dose of LA provides a reduction in disc height comparable with the relatively low dose of condoliase.

Finally, changes in exploratory endpoints after treatment were assessed. At baseline, no leg pain was reported in the 45 mg group. Mean leg pain at screening was 3 mm in the 90 mg group, 14 mm in the 180 mg group and 26 mm in the placebo group. Overall, leg pain remained fairly low throughout the study without any noticeable changes occurring (data not shown).

Baseline mean back pain was 19 mm in the 45 mg group, 44 mm in the 90 mg group, 52 mm in the 180 mg group and 50 mm in the placebo group.

At screening, all treatment groups had a moderate degree of disability in their everyday activities, based on the mean ODI values. No particular trends in changes over time could be discerned (see below).

**Table 10. Changes in VAS (mm) for cLBP in the 4 treatment groups**

| | | **LA 45 mg (N=3)** | **LA 90 mg (N=3)** | **LA 180 mg (N=3)** | **Placebo (N=6)** |
|---|---|---|---|---|---|
| Visit 1 (Screening) | | | | | |
| | n/nmiss | 3/0 | 3/0 | 3/0 | 6/0 |
| | Mean (SD) | 19 (10) | 44 (25) | 52 (18) | 50 (22) |
| Visit 5 (3 months) | | | | | |
| | n/nmiss | 3/0 | 3/0 | 3/0 | 6/0 |
| | Mean (SD) | 22 (8) | 44 (22) | 27 (15) | 40 (23) |
| Visit 6 (6 months) | | | | | |
| | n/nmiss | 2/1 | 3/0 | 3/0 | 5/1 |
| | Mean (SD) | 52 (28) | 44 (6) | 28 (32) | 41 (30) |
| Visit 7 (12 months) | | | | | |
| | n/nmiss | 2/1 | 3/0 | 3/0 | 5/1 |
| | Mean (SD) | 59 (40) | 34 (14) | 61 (26) | 41 (24) |
| n/nmiss = number of patients with evaluable/missing data, SD = standard deviation | | | | | |

**Table 11. Changes in ODI in the 4 treatment groups**

| **ODI** | | **STA363 45 mg (N=3)** | **STA363 90 mg (N=3)** | **STA363 180 mg (N=3)** | **Placebo (N=6)** |
|---|---|---|---|---|---|
| Visit 1 (Screening) | | | | | |
| | n/nmiss | 3/0 | 3/0 | 3/0 | 6/0 |
| | Mean (SD) | 32 (2) | 28 (13) | 25 (10) | 29 (9) |
| Visit 5 (3 months) | | | | | |
| | n/nmiss | 3/0 | 3/0 | 3/0 | 6/0 |
| | Mean (SD) | 35 (10) | 29 (12) | 18 (11) | 26 (6) |
| Visit 6 (6 months) | | | | | |
| | n/nmiss | 2/1 | 3/0 | 3/0 | 5/1 |
| | Mean (SD) | 46 (11) | 32 (9) | 23 (16) | 27 (10) |
| Visit 7 (12 months) | | | | | |
| | n/nmiss | 2/1 | 3/0 | 3/0 | 5/1 |
| | Mean (SD) | 61 (32) | 29 (15) | 28 (11) | 21 (10) |
| n/nmiss = number of patients with evaluable/missing data, SD = standard deviation | | | | | |

Two days after injection of porcine IVDs with LA, lysis of extracellular matrix accompanied by cell death and disappearance were observed. These changes are identical to those seen after injection of typical chemonucleolytics such as chymopapain. Therefore, LA can be labelled as a new chemonucleolytic substance, alongside previously known chemonucleolytics such as chymopapain, condoliase, ozone and ethanol.

The present study shows that LA transforms the porcine NP into connective tissue within one month, as supported by macroscopic and MRI findings. Rapid onset lysis of the NP followed by a more slowly developing fibrosis are typical effects of well-characterised chemonucleolytics. Therefore, LA is by definition a newly identified chemonucleolytic.

LA injection caused a pronounced reduction in lateral flexibility verifying that the newly formed connective tissue stabilised the motion segment. There was a high level of expression of collagen I immunoreactivity in the sclerotized IVDs which may have contributed to the increased stability of the IVD. The sclerosis remained and was even more advanced after 3 months, so it is reasonable to assume that the increased rigidity persisted throughout the experimental period. Remodelling of the AF may also have contributed to reduced flexibility since chondrocytic metaplasia was found in the AF.

Histologically, appearance of cartilaginous tissue was evident after 3 months, and in some IVDs, osteoid islands were seen. Such a tissue transformation suggests that the reduction in flexibility of the lumbar spine may progress over a relatively long time. Another finding was that neovascularization occurred in the fibrotic tissue 3 months after LA administration. It has been suggested that angiogenesis is accompanied by neoinnervation which may generate pain. However, a clear distinction between vasoregulatory and nociceptive afferents has rarely been done. Moreover, angiogenesis and nerve sprouting in degenerated human IVDs seem to be guided by fissures in the AF. Since such fissures were not seen after LA injection, it is possible that the mechanism of invasion of nerves and blood vessels, and the composition of nerve fibres (if they indeed sprout at all), differ from those occurring spontaneously. While there are similarities, it must be recognized that the rapid sclerotization of the IVD produced by LA does not replicate pathologically degenerated IVDs of patients suffering from low back pain. The most important difference in this regard is the absence in the pig studies of some hallmarks of pathological IVD degeneration such as annular fissures and lamellar disorganization.

Although the pig studies include low number of replicates in some groups, this limitation is not likely to affect the conclusions.

It was confirmed that LA transformed the NP into connective tissue in pigs, and MRI observations suggested that this also occurred in patients.

Sclerotization of the IVD makes it more stable resulting in a reduced range of motion which may reduce the probability of herniation relapse. Another effect of the LA-induced sclerosis is prevention of leakage of algogenic molecules and sprouting of nociceptive afferents.

The treatment was safe and tolerable. Several patients reported a relatively shortlasting low back pain (cLBP) after injection but this was also seen in the placebo group. Moreover, with the limitation of the low number of patients, the treatment does not seem to aggravate the cLBP as indicated by the VAS and ODI results.

The MRI results showed that LA induces a reduction in signal intensity in the NP. This may be due to increased breakdown of glycosaminoglycans contributing to hydration of the NP followed by proliferation of connective tissue. As in pigs, the loss of signal intensity in patients was predominantly seen in the periphery of the NP. There was a tendency for a reduction in IVD height after treatment in patients. This effect was more pronounced in porcine IVDs injected with LA, while and in both porcine and human IVDs, IVD width was unchanged. This indirectly but compellingly demonstrates that IVD volume must have decreased. A reduced IVD volume is the goal of all chemonucleolytic treatments since volume reduction causes a decrease in IVD pressure which, in turn, reduces the size of the herniation leading to disappearance or amelioration of symptoms.

The results from the study thus provide proof of concept that the composition for use according to the present invention effectively reduces the disc height and transforms the disc space into connective tissue.

The examples in this application show that the administration of lactic acid results in volume and height reduction, and also a change of the tissue composition of the disc and in a flexion stiffness of the treated IVDs.

While the present invention has been illustrated in the appended drawings and the foregoing description, such illustration is to be considered illustrative or exemplifying and not restrictive; the present invention is not limited to the disclosed embodiments. Other variations to the disclosed embodiments can be understood and effected by those skilled in the art in practicing the claimed invention, from a study of the drawings, the disclosure, and the appended claims. In the appended claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A composition for use in the treatment of intervertebral disc (IVD) herniation, wherein said composition comprises lactic acid, wherein said composition is administered into a disc space comprising the nucleus pulposus (NP) of a herniated intervertebral disc.

2. The composition for use according to claim 1, wherein said composition is administered in an amount effective to increase the concentration of lactic acid in said disc space to at least above 20 mmol/L.

3. The composition for use according to any one of the preceding claims, wherein said composition is administered in an amount effective to dehydrate said herniated IVD.

4. The composition for use according to any one of the preceding claims, wherein said composition is administered in an amount effective to decrease the height of said herniated IVD.

5. The composition for use according to any one of the preceding claims, wherein said composition is administered in an amount effective to initiate fibrosis of said herniated IVD.

6. The composition for use according to any one of the preceding claims, wherein the concentration of lactic acid in said composition is at least 12 mmol/L, preferably from 50 to 12000 mmol/L, more preferably from 100 to 10000 mmol/L, even more preferably from 500 to 5000 mmol/L, most preferably from 800 to 2000 mmol/L.

7. The composition for use according to any one of the preceding claims, wherein said lactic acid is administered by local injection into the disc space comprising the NP of said herniated IVD.

8. The composition for use according to any of claims 1 to 7, wherein the lactic acid is administered in a single dosage within the range of from 2 mg to 1000 mg, preferably from 5 mg to 500 mg, preferably from 10 to 300 mg, more preferably from 20 to 200 mg, more preferably from 90 to 180 mg.

9. The composition for use according to claim 8, wherein said lactic acid is administered at a single occasion in said single dosage.

10. The composition for use according to any one of the preceding claims, wherein said composition is in the form of an aqueous solution comprising said lactic acid.

11. The composition for use according to any of the preceding claims, wherein said composition has a pH below 4.0, preferably below 3.5, more preferably below 3.0.

12. The composition for use according to any of the preceding claims, wherein said composition further comprises a contrast agent.

13. The composition for use according to claim 12, wherein said contrast agent is iodine-containing contrast agent, such as iohexol.

## Patentansprüche

1. Zusammensetzung zur Verwendung bei der Behandlung von Hernien der Bandscheibe (IVD), wobei die Zusammensetzung Milchsäure umfasst, wobei die Zusammensetzung in einen Bandscheibenraum verabreicht wird, der den Nucleus pulposus (NP) einer hernierten Bandscheibe umfasst.

2. Zusammensetzung zur Verwendung nach Anspruch 1, wobei die Zusammensetzung in einer Menge verabreicht wird, die zur Erhöhung der Konzentration von Milchsäure in dem Bandscheibenraum auf mindestens über 20 mmol/l wirksam ist.

3. Zusammensetzung zur Verwendung nach einem der vorhergehenden Ansprüche, wobei die Zusammensetzung in einer Menge verabreicht wird, die zur Dehydratation der hernierten IVD wirksam ist.

4. Zusammensetzung zur Verwendung nach einem der vorhergehenden Ansprüche, wobei die Zusammensetzung in einer Menge verabreicht wird, die zur Verringerung der Höhe der hernierten IVD wirksam ist.

5. Zusammensetzung zur Verwendung nach einem der vorhergehenden Ansprüche, wobei die Zusammensetzung in einer Menge verabreicht wird, die zur Einleitung von Fibrose der hernierten IVD wirksam ist.

6. Zusammensetzung zur Verwendung nach einem der vorhergehenden Ansprüche, wobei die Konzentration von Milchsäure in der Zusammensetzung mindestens 12 mmol/l, vorzugsweise 50 bis 12000 mmol/l, bevorzugter 100 bis 10000 mmol/l, noch bevorzugter 500 bis 5000 mmol/l, besonders bevorzugt 800 bis 2000 mmol/l beträgt.

7. Zusammensetzung zur Verwendung nach einem der vorhergehenden Ansprüche, wobei die Milchsäure durch lokale Injektion in den Bandscheibenraum, der den NP der hernierten IVD umfasst, verabreicht wird.

8. Zusammensetzung zur Verwendung nach einem der Ansprüche 1 bis 7, wobei die Milchsäure in einer Einzeldosis im Bereich von 2 mg bis 1000 mg, vorzugsweise von 5 mg bis 500 mg, vorzugsweise von 10 bis 300 mg, bevorzugter von 20 bis 200 mg, bevorzugter von 90 bis 180 mg verabreicht wird.

9. Zusammensetzung zur Verwendung nach Anspruch 8, wobei die Milchsäure einmalig in der Einzeldosis verabreicht wird.

10. Zusammensetzung zur Verwendung nach einem der vorhergehenden Ansprüche, wobei die Zusammensetzung in Form einer die Milchsäure umfassenden wässrigen Lösung vorliegt.

11. Zusammensetzung zur Verwendung nach einem der vorhergehenden Ansprüche, wobei die Zusammensetzung einen pH unter 4,0, vorzugsweise unter 3,5, bevorzugter unter 3,0 aufweist.

12. Zusammensetzung zur Verwendung nach einem der vorhergehenden Ansprüche, wobei die Zusammensetzung ferner ein Kontrastmittel umfasst.

13. Zusammensetzung zur Verwendung nach Anspruch 12, wobei das Kontrastmittel ein jodhaltiges Kontrastmittel, wie Iohexol ist.

## Revendications

1. Composition pour une utilisation dans le traitement d'une hernie de disque intervertébral (IVD), dans laquelle ladite composition comprend de l'acide lactique, dans laquelle ladite composition est administrée dans un espace discal comprenant le noyau pulpeux (NP) d'un disque intervertébral hernié.

2. Composition pour une utilisation selon la revendication 1, dans laquelle ladite composition est administrée en une quantité efficace pour augmenter la concentration d'acide lactique dans ledit espace discal jusqu'à au moins plus de 20 mmol/L.

3. Composition pour une utilisation selon l'une quelconque des revendications précédentes, dans laquelle ladite composition est administrée en une quantité efficace pour déshydrater ledit IVD hernié.

4. Composition pour une utilisation selon l'une quelconque des revendications précédentes, dans laquelle ladite composition est administrée en une quantité efficace pour diminuer la hauteur dudit IVD hernié.

5. Composition pour une utilisation selon l'une quelconque des revendications précédentes, dans laquelle ladite composition est administrée en une quantité efficace pour initier une fibrose dudit IVD hernié.

6. Composition pour une utilisation selon l'une quelconque des revendications précédentes, dans laquelle la concentration d'acide lactique dans ladite composition est d'au moins 12 mmol/L, préférablement de 50 à 12 000 mmol/L, plus préférablement de 100 à 10 000 mmol/L, encore plus préférablement de 500 à 5 000 mmol/L, le plus préférablement de 800 à 2 000 mmol/L.

7. Composition pour une utilisation selon l'une quelconque des revendications précédentes, dans laquelle ledit acide lactique est administré par injection locale dans l'espace discal comprenant le NP dudit IVD hernié.

8. Composition pour une utilisation selon l'une quelconque des revendications 1 à 7, dans laquelle l'acide lactique est administré en une dose unique dans la gamme de 2 mg à 1 000 mg, préférablement de 5 mg à 500 mg, préférablement de 10 à 300 mg, plus préférablement de 20 à 200 mg, plus préférablement de 90 à 180 mg.

9. Composition pour une utilisation selon la revendication 8, dans laquelle ledit acide lactique est administré en une seule fois dans ladite dose unique.

10. Composition pour une utilisation selon l'une quelconque des revendications précédentes, dans laquelle ladite composition est sous la forme d'une solution aqueuse comprenant ledit acide lactique.

11. Composition pour une utilisation selon l'une quelconque des revendications précédentes, dans laquelle ladite composition a un pH inférieur à 4,0, préférablement inférieur à 3,5, plus préférablement inférieur à 3,0.

12. Composition pour une utilisation selon l'une quelconque des revendications précédentes, dans laquelle ladite composition comprend en outre un agent de contraste.

13. Composition pour une utilisation selon la revendication 12, dans laquelle ledit agent de contraste est un agent de contraste contenant de l'iode, tel que l'iohexol.
